# FASCICULE DE BREVET EUROPEEN

(11) **EP 3 595 627 B1**
(45) Date de publication et mention de la délivrance du brevet: **05.05.2021**
(21) Numéro de dépôt: 18711337.8
(22) Date de dépôt: 16.03.2018
(51) Int. Cl.: A61K 8/49, A61Q 5/00, A61Q 7/00, A61K 8/97, A61K 36/185, A61K 36/31, A61P 17/14

(54) **ASSOCIATION D'EXTRAITS AQUEUX DE CRESSON ET DE CAPUCINE ET D'ATP DANS LE TRAITEMENT DE L'ALOPECIE**
ZUSAMMENSETZUNG AUS WÄSSRIGEN EXTRAKTEN VON KRESSE UND KAPUZINERKRESSE SOWIE ATP IN DER BEHANDLUNG VON ALOPECIA
ASSOCIATION OF AQUEOUS EXTRACTS OF CRESS AND NASTURTIUM AND ATP IN THE TREATMENT OF ALOPECIA

(30) Priorité: 17.03.2017 FR 1752231
(43) Date de publication de la demande: 22.01.2020
(73) Titulaire: Pierre Fabre Dermo-Cosmétique, 92100 Boulogne-Billancourt (FR)
(72) Inventeur: DAUNES-MARION, Sylvie, 31000 Toulouse (FR); LÉVÊQUE, Marguerite, 31400 Toulouse (FR); POIGNY, Stéphane, 31600 Saubens (FR)
(74) Mandataire: Regimbeau
(86) Numéro de dépôt international: PCT/EP2018/056655
(87) Numéro de publication internationale: WO 2018/167265

(56) Documents cités:
- EP-A2- 0 035 919
- CN-A- 1 939 479
- FR-A1- 2 821 271
- Anonymous: "GNPD - Nasturtium & Lemon Shampoo", , 1 novembre 2006 (2006-11-01), XP055394195, Extrait de l'Internet: URL:http://www.gnpd.com/sinatra/recordpage /615444/from_search/1ZHlaLGYrM/?page=1 [extrait le 2017-07-27]
- "LT High Performance Hair Stimulating Shampoo", GNPD, MINTEL, 31 janvier 2014 (2014-01-31), XP002770577,
- Solabia: "Cressatine", , 1 janvier 2016 (2016-01-01), XP055394202, Extrait de l'Internet: URL:http://www.solabia.com/Produto_71,1/Co smetics/Cressatine.html [extrait le 2017-07-27]

## Description

La présente invention concerne une nouvelle association d'un extrait aqueux de cresson et d'un extrait de capucine en association avec de l'ATP, ainsi que l'utilisation de ladite association dans le domaine capillaire plus particulièrement dans le traitement ou la prévention de l'alopécie.

Le follicule pileux est un mini-organe ancré dans la peau jusqu'à l'hypoderme, et dont la fonction principale est la production d'une tige pilaire. Leur distribution est établie au cours de la croissance *in utero* et leur nombre est déterminé génétiquement. Le follicule pileux est une structure dynamique qui produit le cheveu au cours de cycles de croissance et de remodelage de tissus. Ce cycle se décompose en 3 phases :
- Une phase de croissance (anagène), les cellules de la papille dermique (fibroblastes) envoient un signal aux cellules souches du bulbe qui permet leur prolifération. Ces cellules vont se transformer et envelopper la papille dermique pour former la matrice soufrée du cheveu. Elles se divisent et se différencient en kératinocytes, cellules responsables de la structure du cheveu. La durée de cette phase détermine la longueur du cheveu et dépend de la prolifération et de la différenciation des cellules de la matrice à la base du follicule.
- Une phase de régression (catagène), la matrice meurt et de ce fait la papille dermique n'est plus en contact avec cette matrice. Il n'y a plus d'échange entre les cellules. Le follicule et la papille dermique remontent vers l'épiderme.
- Une phase de repos (télogène), les cellules de la papille dermique et du bulbe sont intactes et inactives. Le cheveu tombe. Pour qu'un nouveau cheveu se développe, il faut que le cycle soit réinitié.

Le développement et la croissance du follicule pileux sont influencés par des composés exprimés par la papille dermique, des protéines comme les Wnt et des facteurs de croissance comme ceux des kératinocytes (KGF) ou Epithelium Growth Factor (EGF).

La chevelure se renouvelle donc en permanence et sur les 100 000 à 150 000 cheveux que comporte une chevelure, la majorité est en phase de croissance.

Le terme alopécie désigne la perte partielle ou générale des cheveux. De nombreux facteurs peuvent être impliqués dans l'alopécie comme par exemple les facteurs génétiques, l'âge, le sexe, les maladies, le stress, les problèmes hormonaux, les effets secondaires de médicaments. Il est possible de distinguer plusieurs formes d'alopécie :
- L'alopécie androgénétique, c'est l'alopécie la plus fréquente. La chute précoce des cheveux survient chez des sujets prédisposés génétiquement et elle atteint en particulier les hommes. Elle se manifeste par une diminution du volume des cheveux, voire une calvitie et touche 50% des hommes âgés de plus de 50 ans ;
- L'alopécie post-ménopausique, il s'agit de la plus fréquente cause de la calvitie chez la femme. Chez la femme, la chute des cheveux est plus diffuse et étendue que chez l'homme. L'alopécie diffuse féminine est un désordre qui démarre souvent à la ménopause et qui concerne environ 40% des femmes âgées de plus de 70 ans. Le terme diffus illustre que, contrairement à l'homme, la perte des cheveux concerne la totalité du cuir chevelu, de manière homogène ;
- L'alopécie aiguë, elle peut être liée à un traitement par chimiothérapie, un stress, des carences alimentaires importantes, une carence en fer, des troubles hormonaux ;
- L'alopécie cicatricielle, elle peut être provoquée par des problèmes de peau (tumeur, brûlure, pelade), une irradiation aiguë, au lupus érythémateux ou des parasites (teigne, lichen) ;
- L'alopécie areata, elle semble être d'origine auto-immune et se caractérise par une atteinte en plaques plus ou moins larges et à un ou plusieurs endroits ;
- L'alopécie congénitale, rare, elle correspond à, une absence de racines ou à des anomalies du cheveu (mutations).

L'alopécie est essentiellement liée à une perturbation du renouvellement capillaire qui entraine, dans un premier temps, l'accélération de la fréquence des cycles aux dépens de la qualité des cheveux puis de leur quantité. Le phénomène le plus fréquent est une réduction du cycle de croissance (phase anagène) dû à un arrêt de la prolifération cellulaire. La conséquence est une induction prématurée de la phase catagène et un nombre plus important de follicules pileux en phase télogène et donc une chute plus importante des cheveux. Pour lutter contre l'alopécie, il est donc nécessaire de relancer le cycle pilaire, par exemple, en activant la phase anagène.

Pour combattre cette chute de cheveux, il a été utilisé plusieurs procédés. Certains tendent à accroitre l'irrigation du sang par irradiation ou massage, d'autres méthodes font usage de traitement externe ou par injections locales de produits contenant des substances chimiques stimulantes, toniques et bactéricides comme l'acide salicylique, la résorcine, des alcools, des sulfamides, l'acétylcholine, la choline, la bétaïne, l'acide pantothénique, etc. D'autres procédés s'efforcent d'influencer l'échange cellulaire à l'aide des vitamines F ou du complexe B, des hormones de diverses provenances, de la cholestérine, de la lécithine ou des produits de la kératine. Le soufre, les sels de thallium ou de mercure ont été proposés pour leur action désinfectante et curative. Enfin, la cortisone et l'ACTH (hormone adrénocorticotrope) sont connus par le fait qu'ils freinent la chute des cheveux et produisent leur régénération, mais seulement pendant leur administration, qui se fait pour d'autres fins et leur action sur les cheveux disparait après quelques semaines, lorsque le traitement est arrêté. Au cours des dernières années, l'usage de l'adénosine triphosphate (ATP) a été dévoilé dans le traitement antichute, comme source d'énergie de la papille folliculaire pour prolonger la vie des cheveux. Dans la cellule, l'ATP est la source d'énergie principale, le précurseur des cofacteurs NAD⁺ et coenzyme A. De plus, l'ATP est un intermédiaire dans la communication cellulaire. Il joue un rôle essentiel dans de nombreux tissus par son action sur les récepteurs purinergiques P₂Y et P₂X. Dans le follicule pileux humain, l'expression des récepteurs P₂Y et P₂X a été mise en évidence dans des compartiments distincts pendant la phase anagène, suggérant un rôle de l'ATP dans la physiologie du follicule pileux FR 2 821 271 A1 concerne une composition à mode d'administration oral destinée à empêcher ou à ralentir la chute anormale des cheveux et à améliorer la solidité des ongles. La composition peut contenir en outre d'autres extraits de plantes, notamment des extraits de cresson et/ ou de capucine.

De façon surprenante, les inventeurs ont découvert que l'association ATP avec un extrait de cresson, en particulier un extrait aqueux de cresson, en particulier un extrait aqueux de feuilles de cresson, et un extrait de capucine, en particulier un extrait aqueux de capucine, induisait une synergie d'action sur HGF (Hepatocyte Growth Factor) et sur KGF (Keratinocyte Growth Factor), détaillé dans l'exemple 1.

Comme produit convenant particulièrement bien à l'invention, on peut citer la matière commerciale Cressatine® vendue par la société Solabia. La Cressatine® est un actif obtenu par l'extraction aqueuse des parties aériennes de cresson (*Nasturtium officinale*) et de capucine (*Trapaeolum majus*), stabilisé avec de la glycérine végétale, cet actif contient une quantité équivalente de cresson et de capucine. Cet actif a été conçu pour faciliter la croissance du cheveu et le fortifier dès la racine. Il participe à l'initiation de la régénération du cheveu *via* la voie Wnt. Enfin il prolonge la durée de la phase de croissance grâce à la voie Wnt.

Dans un premier mode de réalisation, l'invention concerne une association comprenant un extrait de cresson, plus particulièrement un extrait aqueux de cresson, plus particulièrement encore un extrait aqueux de parties aériennes de cressons, et encore plus particulièrement encore un extrait aqueux de feuilles de de cresson ; un extrait de capucine, particulièrement un extrait aqueux de capucine, plus particulièrement encore un extrait aqueux de parties aériennes de capucine, et de l'ATP.

L'ATP ou adénosine 5'-triphosphate, est un nucléotide de la famille des purines servant à emmagasiner et à transporter de l'énergie (les purines sont des bases azotées). Il est constitué de l'adénine (une base azotée), du ribose (un sucre avec cinq atomes de carbone) et de trois groupes phosphates unis les uns aux autres par deux liaisons pyrophosphates à haut potentiel énergétique. L'ATP est très soluble dans l'eau. Il demeure relativement stable en solution aqueuse pour des pH compris entre 6,8 et 7,4.

Dans le cadre de l'invention on pourra utiliser de l'ATP sous sa forme salifiée ou non, hydratée ou non.

De manière préférentielle, on utilisera un sel d'ATP, tel qu'un sel avec le Na, Ca, Mg par exemple, hydraté ou non.

On pourra utiliser ainsi particulièrement un sel monosodique ou disodique, monohydraté ou dihydraté.

En particulier l'ATP pourra être l'hydrate disodique d'adenosine 5'-triphosphate.

Dans un deuxième mode de réalisation, l'invention vise une association selon le mode de réalisation 1, pour son utilisation dans la prévention et/ou le traitement de l'alopécie.

Dans un troisième mode de réalisation, l'invention vise une association pour son utilisation selon le mode de réalisation 1, dans laquelle l'alopécie est choisie dans le groupe constitué par l'alopécie androgénétique, l'alopécie post-ménopausique, l'alopécie aiguë et l'alopécie aerata.

Dans un quatrième mode de réalisation, l'invention vise une utilisation cosmétique de l'association selon le mode de réalisation 1, pour promouvoir la croissance capillaire.

Dans un cinquième mode de réalisation, l'invention vise une composition cosmétique ou dermatologique comprenant à titre de principe actif anti-alopécie une association selon le mode de réalisation 1, avec au moins un excipient cosmétiquement ou dermatologiquement acceptable.

Dans un sixième mode de réalisation, l'invention vise une composition selon le mode de réalisation 5, caractérisée en ce qu'elle comprend 0,01% à 0,5% en poids d'un extrait de cresson, plus particulièrement un extrait aqueux de cresson, par rapport au poids total de la composition.

Dans un septième mode de réalisation, la présente invention vise une composition selon l'un des modes de réalisation 5 ou 6, caractérisée en ce qu'elle comprend 0,01% à 0,5% en poids d'un extrait de capucine, plus particulièrement un extrait aqueux de capucine, par rapport au poids total de la composition.

Dans un huitième mode de réalisation, l'invention vise une composition selon l'un des modes de réalisation 5 à 7, caractérisée en ce qu'elle comprend 0,1% à 0,5% en poids d'ATP par rapport au poids total de la composition.

Dans un neuvième mode de réalisation, l'invention vise une composition selon l'un quelconque des modes de réalisation 5 à 8, caractérisée en ce qu'elle comprend en outre un autre principe actif anti-alopécie.

Dans un dixième mode de réalisation, l'invention vise une composition selon l'un quelconque des modes de réalisation 5 à 9, caractérisée en ce qu'elle se présente sous une forme propre à une application topique.

Dans un onzième mode de réalisation, l'invention vise une utilisation cosmétique d'une composition cosmétique selon l'un quelconque des modes de réalisation 5 à 10 pour promouvoir la croissance capillaire.

Dans un douzième mode de réalisation, l'invention vise une composition dermatologique selon l'un des modes de réalisation 5 à 10, pour son utilisation dans la prévention et/ou le traitement de l'alopécie.

Dans un treizième mode de réalisation, l'invention vise encore un procédé de soin cosmétique des cheveux destiné à améliorer l'esthétique des cheveux en favorisant la pousse capillaire, caractérisé en ce qu'il consiste à appliquer sur les cheveux et le cuir chevelu une quantité efficace d'une association comprenant un extrait de cresson, plus particulièrement un extrait aqueux de cresson ; un extrait de capucine, plus particulièrement un extrait aqueux de capucine, et de l'ATP selon le premier mode de réalisation ou d'une composition cosmétique selon l'un des modes de réalisation 5 à 10, à laisser celle-ci au contact des cheveux, et éventuellement à rincer les cheveux. Plus particulièrement l'extrait aqueux de cresson est un extrait aqueux de parties aériennes de cressons, et encore plus particulièrement encore un extrait aqueux de feuilles de cresson. Particulièrement, l'extrait de capucine est un extrait aqueux de parties aériennes de capucine, en particulier de feuilles.

Par l'amélioration de l'esthétique des cheveux, on entend en particulier que la chevelure est plus couvrante et/ou que le cuir chevelu est moins visible.

La présente invention a donc pour l'objet une association d'un extrait de cresson, plus particulièrement un extrait aqueux de cresson et d'un extrait de capucine, plus particulièrement un extrait aqueux de capucine, en association avec de l'ATP.

L'extrait aqueux de cresson est préférentiellement un extrait de feuilles de cresson. Le cresson de fontaine ou Nasturtium officinale appartient à la famille de Brassicaceae. Le cresson de fontaine est une plante vivace qui forme des pousses rampantes au fond de l'eau, puis des tiges creuses qui se dressent hors de l'eau à l'extrémité des rameaux. Les tiges sont étalées, voire couchées sur le sol ou sur les plans d'eau. Elles peuvent dépasser deux mètres de long. Le cresson présente un taux record de fer et de calcium ainsi que beaucoup de vitamine C. Semé en pépinière dans un terreau maintenu à l'humidité ou en pleine terre, le cresson peut se développer, être repiqué et être récolté environ trois mois après le semis et un mois après le repiquage.

L'extrait de cresson utilisé selon l'invention est un extrait aqueux de cresson, préférentiellement un extrait aqueux de parties aériennes de cresson, plus particulièrement un extrait aqueux de tiges et/ou feuilles de cresson. Plus particulièrement encore, un extrait convenable pour l'invention est bien connu de l'homme du métier et référencé sous le N° CAS 84775-70-2, il est composé d'extrait aqueux de feuilles et tiges de cresson de fontaine.

L'extrait aqueux de capucine est préférentiellement un extrait aqueux de parties aériennes de capucine, plus particulièrement de feuilles de capucines.

La capucine (Tropaeolum majus) est une plante herbacée annuelle ou vivace de la famille des Tropaéolacées et du genre Tropaeolum. Cette plante ornementale est comestible. La capucine est riche en vitamine C, notamment son feuillage elle est d'ailleurs souvent récoltée sans les fleurs de mai à octobre. Le suc de la plante à l'état frais a des propriétés antiseptique, stimulante, expectorante et diurétique. La plante contient un glucosinolate (la glucotropaeoline) et une enzyme, la myrosinase qui hydrolyse le premier en thiocyanates, isothiocyanates et nitriles, molécules présentant une activité toxique contre les insectes herbivores

Les graines, les feuilles et les fleurs sont utilisées, fraiches ou séchées en phytothérapie.

L'extrait de capucine utilisé selon l'invention est un extrait aqueux de capucine, préférentiellement un extrait aqueux de parties aériennes de capucines, plus particulièrement un extrait aqueux de fleurs, tiges et/ou feuilles de capucine. Plus particulièrement encore, un extrait de capucine convenable pour l'invention est bien connu de l'homme du métier et référencé sous le N° CAS 84625-49-0, il est composé d'extrait aqueux de parties aériennes de capucines, en particulier un extrait de fleurs, feuilles et/ou tiges, plus particulièrement de feuilles de capucine.

Une produit associant les deux extraits, de capucine et de cresson, particulièrement préféré pour réaliser l'association selon l'invention est la Cressatine®, commercialisé par la société Solabia®. Comme indiqué supra, un tel produit est disponible dans le commerce et correspond à mélange d'un extrait aqueux de feuilles de cresson (Nasturtium officinale) et d'un extrait aqueux de feuilles de capucine (Tropaeolum majus), stabilisé avec de la glycérine végétale et titré en soufre (200 mg/kg).

Dans un mode de réalisation particulier l'association selon l'invention est une association consistant en un extrait aqueux de cresson, un extrait aqueux de capucine et de l'ATP.

Préférentiellement, la présente invention a pour objet l'utilisation de l'association d'un extrait de cresson, particulièrement un extrait aqueux de cresson et d'un extrait de capucine, plus particulièrement un extrait aqueux de capucine, en association avec de l'ATP dans la prévention et/ou le traitement de l'alopécie. Plus particulièrement l'extrait aqueux de cresson est un extrait aqueux de parties aériennes de cressons, et encore plus particulièrement encore un extrait aqueux de feuilles de cresson. Particulièrement, l'extrait de capucine est un extrait aqueux de parties aériennes de capucine, en particulier de feuilles.

Plus particulièrement, la présente invention a pour l'objet l'utilisation de l'association d'extraits de cresson et de capucine, plus particulièrement d'extraits aqueux de cresson et de capucine, préférentiellement stabilisé avec de la glycérine végétale, et d'ATP pour la prévention et/ou le traitement de l'alopécie androgénétique, l'alopécie post-ménopausique, l'alopécie aiguë ou l'alopécie aerata. Avantageusement, la présente invention a pour l'objet l'utilisation de l'association d'un extrait de cresson , préférentiellemet un extrait aqueux de cresson et d'un extrait de capucine, préférentiellement un extrait aqueux de capucine, en association avec de l'ATP pour la prévention et/ou le traitement de l'alopécie androgénétique. Plus particulièrement l'extrait aqueux de cresson est un extrait aqueux de parties aériennes de cressons, et encore plus particulièrement encore un extrait aqueux de feuilles de cresson. Particulièrement, l'extrait de capucine est un extrait aqueux de parties aériennes de capucine, en particulier de feuilles.

La présente invention a également pour objet l'utilisation de l'association d'un extrait de cresson, particulièrement un extrait aqueux de cresson et d'un extrait de capucine, particulièrement un extrait aqueux de capucine, en association avec de l'ATP pour promouvoir la croissance capillaire. Plus particulièrement l'extrait aqueux de cresson est un extrait aqueux de parties aériennes de cressons, et encore plus particulièrement encore un extrait aqueux de feuilles de cresson. Particulièrement, l'extrait de capucine est un extrait aqueux de parties aériennes de capucine, en particulier de feuilles.

L'invention concerne également l'utilisation de l'association d'un extrait de cresson, particulièrement un extrait aqueux de cresson et d'un extrait de capucine, particulièrement un extrait aqueux de capucine, en association avec de l'ATP pour son utilisation dans la prévention et/ou le traitement de l'alopécie, sous une forme adaptée à une administration par voie topique et/ou par voie orale. Plus particulièrement l'extrait aqueux de cresson est un extrait aqueux de parties aériennes de cressons, et encore plus particulièrement encore un extrait aqueux de feuilles de cresson. Particulièrement, l'extrait de capucine est un extrait aqueux de parties aériennes de capucine, en particulier de feuilles.

Dans un autre aspect, la présente invention concerne l'utilisation cosmétique ou dermatologique de l'association d'un extrait de cresson, particulièrement un extrait aqueux de cresson et d'un extrait de capucine, particulièrement un extrait aqueux de capucine, en association avec de l'ATP pour limiter la chute des cheveux/poils et/ou promouvoir leur croissance et/ou augmenter la densité des follicules pileux. Plus particulièrement l'extrait aqueux de cresson est un extrait aqueux de parties aériennes de cressons, et encore plus particulièrement encore un extrait aqueux de feuilles de cresson. Particulièrement, l'extrait de capucine est un extrait aqueux de parties aériennes de capucine, en particulier de feuilles.

Dans un autre aspect, la présente invention concerne également un procédé cosmétique ou dermatologique pour limiter la chute des cheveux/poils et/ou promouvoir leur croissance et/ou augmenter la densité des follicules pileux comprenant une étape d'administration de l'association d'un extrait de cresson, particulièrement un extrait aqueux de cresson et d'un extrait de capucine, particulièrement un extrait aqueux de capucine, en association avec de l'ATP à un individu. Plus particulièrement l'extrait aqueux de cresson est un extrait aqueux de parties aériennes de cressons, et encore plus particulièrement encore un extrait aqueux de feuilles de cresson. Particulièrement, l'extrait de capucine est un extrait aqueux de parties aériennes de capucine, en particulier de feuilles.

La présente invention peut être utilisée en association avec des traitements connus de l'alopécie ou des molécules connues pour favoriser la croissance des cheveux/poils ou limiter leurs chutes comme par exemple le finastéride, le minoxidil ou bénéficier à un individu qui a subi ou va subir une micro greffe.

La présente invention peut également être utilisée en association avec des composés utiles pour une bonne structure du cheveu, comme par exemple des protéines soufrées, de la vitamine B6 et différents minéraux comme le zinc et/ou le magnésium.

Par « cheveux/poils », on entend la chevelure, les poils, les sourcils, les cils et/ou le pelage. Préférentiellement, par cheveux/poils, on entend les cheveux.

Par « alopécie » on entend la perte totale ou partielle des cheveux par exemple liée à la réduction de la croissance capillaire et/ou à l'accélération de la chute des cheveux/poils. Ce terme inclut mais ne se limite pas à l'alopécie androgénétique, l'alopécie post-ménopausique, l'alopécie aiguë, l'alopécie cicatricielle, l'alopécie aerata, l'alopécie congénitale. Les conséquences de l'alopécie sont une absence temporelle ou définitive et partielle ou totale des cheveux.

Par le terme « traiter l'alopécie », on entend l'arrêt de l'alopécie, la réduction de l'alopécie et/ou l'atténuation de l'alopécie. Ainsi « traiter l'alopécie » comprend limiter la chute des cheveux, promouvoir la croissance des cheveux, augmenter la densité des follicules pileux et/ou réguler les phases du cycle du follicule pileux.

Par le terme « prévenir », on entend diminuer le risque d'apparition de l'alopécie, ou de ralentir la progression de l'alopécie chez un mammifère, préférentiellement l'homme qui est susceptible de développer une alopécie.

Par le terme « limiter », on entend freiner, réduire, diminuer et ou stopper.

Par le terme « promouvoir », on entend augmenter, accroitre, favoriser, amplifier et/ou accélérer.

La présente invention concerne aussi une composition cosmétique ou dermatologique pour la prévention et ou le traitement de l'alopécie comprenant une association comprenant un extrait de cresson et de capucine, particulièrement un extrait aqueux de cresson et de capucine, préférentiellement stabilisé avec de la glycérine végétale, et de l'ATP avec un excipient cosmétiquement ou dermatologiquement acceptable, particulièrement acceptable pour une application topique et/ou pour une voie orale. Plus particulièrement l'extrait aqueux de cresson est un extrait aqueux de parties aériennes de cressons, et encore plus particulièrement encore un extrait aqueux de feuilles de cresson. Particulièrement, l'extrait de capucine est un extrait aqueux de parties aériennes de capucine, en particulier de feuilles.

Un autre objet de la présente invention concerne une composition dermatologique pour son utilisation dans le traitement de l'alopécie, et notamment l'alopécie androgénétique comprenant en tant que principe actif l'association selon l'invention. Dans un mode de réalisation particulier, l'association selon l'invention est le seul principe actif anti-alopécie de la composition.

Dans la présente invention, on entend désigner par « cosmétiquement ou dermatologiquement acceptable » ce qui est utile dans la préparation d'une composition cosmétique ou dermatologique, qui est généralement sûre, non toxique et ni biologiquement ni autrement non souhaitable et qui est acceptable pour une utilisation cosmétique, notamment par application topique et/ou par voie orale.

Les compositions cosmétiques ou dermatologiques selon l'invention pourront se présenter sous les formes qui sont habituellement connues pour une administration topique, c'est-à-dire notamment les lotions, les shampoings, les baumes, les mousses, les gels, les dispersions, les émulsions, les sprays, les sérums, les masques ou les crèmes, avec des excipients permettant notamment une pénétration afin d'améliorer les propriétés et l'accessibilité des principes actifs.

Avantageusement les compositions selon l'invention peuvent se présenter sous les formes qui sont habituellement connues pour une administration topique sur les cheveux et le cuir chevelu, c'est-à-dire notamment un shampoing, un après shampoing, une crème capillaire, une lotion capillaire ou un spray sans rinçage.

On distingue ainsi des produits formulés pouvant être rincés et d'autres non.

L'invention vise ainsi des compositions cosmétiques ou dermatologiques selon l'un des modes de réalisation de la présente invention, caractérisées en ce qu'elles se présentent sous une forme propre et adaptée à une application topique.

Avantageusement une composition topique cosmétologique et/ou dermatologique selon l'invention est caractérisée en ce qu'elle se présente sous forme de trois phases distinctes, destinées à être mélangées extemporanément de façon à obtenir une émulsion et/ou microémulsion, lesdites phases étant une phase solide sous une forme pulvérulente, une phase aqueuse et une phase lipophile.

Dans une telle configuration, les éléments de l'association selon l'invention, l'extrait de cresson, en particulier l'extrait aqueux de cresson, l'extrait de capucine, en particulier l'extrait aqueux de capucine et l'ATP sont compris dans des compartiments physiquement séparés. En particulier, l'extrait aqueux de cresson et l'extrait aqueux de capucin pourront être présents dans le même compartiment de la composition et l'ATP sera compris dans un autre compartiment comprenant la phase solide pulvérulente, physiquement séparés des extraits aqueux. Plus particulièrement l'extrait aqueux de cresson est un extrait aqueux de parties aériennes de cressons, et encore plus particulièrement encore un extrait aqueux de feuilles de cresson. Particulièrement, l'extrait de capucine est un extrait aqueux de parties aériennes de capucine, en particulier de feuilles.

Dans une telle composition topique, la phase solide sous forme de poudre est constituée d'actifs et/ou d'excipients dermatologiques ou cosmétiques choisis dans le groupe constitué par : silice amorphe, cyclodextrine(beta), glycyrrhetinate stearyl, cocoyl glutamate sodium, lactose, ATP, des tensioactifs, seuls ou en mélange. Les tensioactifs peuvent être choisis parmi les dérivés anioniques et plus particulièrement le lauryl sulfate de magnésium, le lauryl sulfosuccinate et le cocoyliséthionate de sodium, les dérivés non ioniques et plus particulièrement le lauryl alcool éthoxylé et les nonylphénols ; les dérivés cationiques et plus particulièrement le bromure de cétyldiméthylbenzyl ammonium et le chlorure de cétyltriméthyl ammonium ; les dérivés amphotères et plus particulièrement la lauryl bétaine et les dérivés d'imidazoline et leurs mélanges.

La phase solide peut comprendre des émulsionnants et plus particulièrement le lactosérum et la poudre de lécithine d'oeuf.

Cette phase solide peut en particulier comprendre des principes actifs peu stables en solution et/ou difficilement solubilisables. C'est ainsi que préférentiellement, dans cette phase solide pulvérulente, est compris l'ATP des compositions selon l'invention.

La phase lipophile est composée d'au moins une huile végétale ou animale ou minérale, seules ou en mélange, cosmétiquement ou dermatologiquement acceptable. L'huile végétale peut être choisie dans le groupe comprenant l'huile d'amande douce, l'huile d'avocat, l'huile de coco, l'huile de germe de blé, l'huile de maïs, l'huile d'olive, l'huile de palme, l'huile de sésame, l'huile de soja, l'huile d'argan, l'huile d'onagre, l'huile de bourrache, l'huile de pépin de courge hydrolysée, les huiles essentielles et le cires végétales, ainsi que leurs mélanges. Les huiles essentielles peuvent être choisies parmi les huiles essentielles d'orange douce ou de lavande par exemple.

L'huile minérale peut être une huile cosmétique ou dermatologique choisie dans la série des paraffines, ou huile de vaseline, huile de silicone ; par exemple le dodécane ou le tetradécane.

La phase lipophile peut aussi comprendre une phase grasse/huileuse comprenant ou constituée d'esters gras, tels que des esters gras de vitamine A, en particulier le tocophéryl acétate.

La phase lipophile peut aussi comprendre des huiles animales et plus particulièrement d'huile de castor, d'huile de tortue, d'huile de vison, d'huiles de poissons et de cires animales.

Selon la présente invention, cette phase lipidique peut également contenir des dérivés lipophiles stables et plus particulièrement des colorants, des filtres solaires, des antioxydants, des conservateurs et également des principes actifs.

La phase aqueuse peut contenir des dérivés hydrosolubles stables et plus particulièrement des colorants, des conservateurs, et également des principes actifs hydrosolubles.

La phase aqueuse peut comprendre les composés suivants : alcool éthylique 96%, chlorhydrate pyridoxine, hesperidine methyl chalcone, eau purifiée, niacinamide, extrait hydroalc de racines de pfaffia, extrait aqueux de cresson, extrait aqueux de capucine, colorants, utilisés seuls ou en mélange.

La phase aqueuse comprend particulièrement l'association de l'extrait aqueux de cresson et l'extrait aqueux de capucine tel qu'évoquée supra.

Selon la présente invention, la formulation triphasique contient 0,1 à 10 % en poids de la phase solide, 1 à 50 % en poids de la phase lipophile et 10 à 90 % en poids de la phase aqueuse.

Enfin, selon une formulation préférée, la composition contient de préférence 1 à 5 % en poids de la phase solide, 10 à 25 % en poids de la phase lipophile et 50 à 70 % de la phase aqueuse.

L'invention vise ainsi dans un de ses modes de réalisation une composition topique cosmétologique et/ou dermatologique, caractérisée en ce qu'elle se présente sous forme de trois phases distinctes, destinées à être mélangées extemporanément de façon à obtenir une émulsion et/ou microémulsion, lesdites phases étant :
- une phase solide contant de l'ATP sous une forme pulvérulente,
- une phase aqueuse contenant un extrait aqueux de cresson et un extrait aqueux de capucine et
- une phase lipophile.

Une telle composition triphasique est particulièrement adaptée pour son utilisation dans le traitement préventif ou curatif de l'alopécie.

Une association ou une composition selon l'invention est particulièrement adaptée pour être mise en œuvre dans un procédé de traitement cosmétique du cuir chevelu permettant d'obtenir une chevelure plus couvrante comprenant l'application de l'association ou de la composition sur le cuir chevelu, particulièrement entre 1 et 3 fois par semaine.

Préférentiellement l'application d'une association ou d'une composition selon l'invention n'est pas suivie de rinçage.

Une composition triphasique selon l'invention telle que décrite plus avant est particulièrement adaptée pour être mise en œuvre dans un procédé de traitement cosmétique du cuir chevelu permettant d'obtenir une chevelure plus couvrante comprenant l'application de la composition sur le cuir chevelu, particulièrement entre 1 et 3 fois par semaine.

Préférentiellement l'application d'une association ou d'une composition selon l'invention n'est pas suivie de rinçage.

Les compositions selon l'invention peuvent également être administrées par voie orale. Dans ce cas, les formes unitaires d'administration appropriées comprennent les formes adaptées pour une voie orale, telles que les comprimés, les gélules, les poudres, les granules et les solutions ou suspensions orales.

Ces compositions contiennent généralement, outre les composés de l'association selon la présente invention, un milieu physiologiquement acceptable, en général à base d'eau ou de solvant, par exemple des alcools, des éthers ou des glycols. Elles peuvent également contenir des agents tensioactifs, des agents complexant, des conservateurs, des agents stabilisants, des émulsifiants, des épaississants, des gélifiants, des humectants, des émollients, des oligo-éléments, des huiles essentielles, des parfums, des colorants, des agents hydratants ou des eaux thermales, etc.

Avantageusement, les compositions selon la présente invention comprendront 0,01 à 1% en poids, de préférence 0,01 à 0,5% en poids, de manière préférée 0,02 à 0,1% en poids, de manière encore préférée 0,02 à 0,05% en poids d'extrait de cresson, particulièrement d'extrait aqueux de cresson, par rapport au poids total de la composition. D'une manière préférée, la composition comprendra environ 0,02% en poids d'extrait de cresson, particulièrement d'extrait aqueux de cresson, par rapport au poids total de la composition.

Avantageusement, les compositions selon la présente invention comprendront 0,01 à 1% en poids, de préférence 0,01 à 0,5% en poids, de manière préférée 0,02 à 0,1% en poids, de manière encore préférée 0,02 à 0,05% en poids d'extrait de capucine, particulièrement d'extrait aqueux de capucine, par rapport au poids total de la composition. D'une manière préférée, la composition comprendra environ 0,02% en poids d'extrait de capucine, particulièrement d'extrait aqueux de capucine par rapport au poids total de la composition.

Plus particulièrement l'extrait aqueux de cresson est un extrait aqueux de parties aériennes de cressons, et encore plus particulièrement encore un extrait aqueux de feuilles de cresson. Particulièrement, l'extrait de capucine est un extrait aqueux de parties aériennes de capucine, en particulier de feuilles.

D'une façon préférée, les compositions selon l'invention comprendront la même teneur en pourcentage d'extrait de cresson et de capucine, particulièrement d'extrait aqueux de cresson et de capucine, par rapport au poids total de la composition.

Avantageusement, les compositions selon la présente invention comprendront 0,1 à 1% en poids, de préférence 0,1 à 0,5% en poids, de manière préférée 0,2 à 0,5% en poids, de manière encore préférée 0,25 à 0,4% en poids d'ATP par rapport au poids total de la composition. D'une manière préférée, la composition comprendra environ 0,3% en poids d'ATP par rapport au poids total de la composition.

De manière particulière, lorsque l'on exprime les quantités d'extrait de cresson, d'extrait de capucine et d'ATP dans l'association ternaire selon l'invention, en parts, cette association comprend entre 0,5 et 2 parts d'extrait de capucine, entre 0,5 et 2 parts d'extrait de cresson et entre 5 et 20 parts d'ATP. De manière préférée, l'association selon l'invention comprend, en parts, 1 part d'extrait de capucine, 1 part d'extrait de cresson et 15 parts d'ATP. Plus particulièrement il s'agit d'extraits aqueux de cresson et de capucine. Plus particulièrement l'extrait aqueux de cresson est un extrait aqueux de parties aériennes de cressons, et encore plus particulièrement encore un extrait aqueux de feuilles de cresson. Particulièrement, l'extrait de capucine est un extrait aqueux de parties aériennes de capucine, en particulier de feuilles.

La texture légère de ladite composition permet en outre une pénétration optimale sans graisser les cheveux. Dès les premières applications, la chevelure retrouve force et vitalité.

La composition selon l'invention permet d'enrayer la chute du cheveu, d'en prolonger son cycle. Le capital cheveu est préservé, en quantité et en qualité.

Dans un mode de réalisation particulier, les compositions cosmétiques ou dermatologiques selon l'invention comprennent au moins un autre principe actif anti-alopécie.

Un autre objet de la présente invention vise l'utilisation cosmétique de l'association selon la présente invention ou de cette composition cosmétique selon l'invention pour promouvoir la croissance capillaire et/ou obtenir une chevelure plus couvrante. L'utilisation cosmétique de l'association selon la présente invention ou la composition cosmétique selon l'invention est plus particulièrement destinée à réguler le cycle pilaire et favoriser la régénération folliculaire.

La présente invention concerne également un procédé de soin cosmétique des cheveux destiné à améliorer l'esthétique des cheveux en favorisant la pousse capillaire et/ou obtenir une chevelure plus couvrante caractérisé en ce qu'il consiste à appliquer sur les cheveux et le cuir chevelu une quantité efficace d'une association selon l'invention ou d'une composition cosmétique selon l'invention, à laisser celle-ci au contact des cheveux, et éventuellement à rincer les cheveux.

De telles compositions peuvent être fabriquées selon des procédés bien connus de l'homme du métier.

Les exemples qui suivent sont destinés à illustrer certains modes de réalisation particuliers de l'invention et représentent en particulier certaines compositions utilisables pour la mise en œuvre de l'invention. Les excipients mentionnés dans les exemples de composition ne sont donnés qu'à titre illustratif, et il est à la portée de l'homme du métier d'en substituer d'autres.

### Exemple 1 : test pharmacologique d'un extrait aqueux de cresson et de capucine, associé ou non avec de l'ATP

Le but de cette étude est d'évaluer les effets de l'association d'un extrait aqueux de cresson et d'un extrait aqueux de capucine, associé à de l'ATP sur la libération de 2 marqueurs de la phase anagène HGF et KGF (Hepatocyte or Keratinocyte Growth Factor) libérés par les cellules des papilles dermiques. Ces deux facteurs de croissance ont une activité stimulatrice sur la croissance du cheveu en activant des voies de signalisation spécifiques.

Les études ont été réalisées sur des cellules de la papille dermique issues de follicules pileux humains en provenance de trois donneurs différents. Les cellules sont ensemencées dans des plaques de 24 puits et mises en culture pendant 24 h avec les compléments nécessaires. Les cellules sont ensuite traitées pendant 24 h avec les produits, c'est-à-dire soit avec l'ATP (30µM), soit avec de la Cressatine® c'est-à-dire un extrait aqueux de cresson et un extrait aqueux de capucine (140 µg/ml), ou bien encore avec l'association ATP-Cressatine® aux mêmes concentrations que précédemment. Les surnageants étaient collectés pour l'analyse ultérieure des marqueurs HGF et KGF. HGF est mesuré à l'aide du set HCCBP1MAG-58K de EMD Millipore et KGF est mesuré en utilisant le set KGF/FGF-7 DuoSet ELISA DY251 de R&D Systems.

Les résultats sont résumés dans les tableaux suivants.

### Libération de HGF :

L'ATP seul à 30µM ne module pas significativement la production de HGF (Tableaux 1). Les extraits aqueux de cresson et de capucine, à 140 µg/ml induisent une augmentation significative de la production de HGF (p<0.01 versus contrôle), cette élévation atteint 66%. Quand ils sont associés, l'ATP à 30µM et la Cressatine® à 140 µg/ml induisent une augmentation significative et très marquée de la libération de HGF, cette augmentation atteint 133%. L'induction obtenue avec cette combinaison est significativement plus importante que celle obtenue avec la Cressatine® seule montrant ainsi l'effet synergique de ces composés.

**Tableau 1 : libération de HGF par les cellules de la papille dermique issues de follicules pileux humains. Les résultats sont exprimés en valeurs moyennées des 3 donneurs ± sem (sem = écart standard à la moyenne).**

| | HGF (pg/ml) | sem | Comparaison, test Dunnett | |
|---|---|---|---|---|
| | | | Vs control | Vs Cressatine® |
| Contrôle | 80 | 17 | - | - |
| ATP | 92 | 21 | P=NS | - |
| Cressatine® | 120 | 25 | P<0,01 | - |
| ATP + Cressatine® | 167 | 37 | P<0,01 | P<0,01 |

| | | | | |
|---|---|---|---|---|
| Cressatine® : extrait aqueux de cresson et de capucine. | | | | |

### Libération de KGF :

L'ATP seul à 30µM est capable de stimuler significativement la production de KGF (tableau 2) et induit une sécrétion accrue de 50%. L'extrait aqueux de cresson et de capucine, à 140 µg/ml seul n'induit pas d'augmentation significative de cette libération. En revanche l'association de ces composés aux mêmes concentrations induit une libération importante et significative de KGF, cette production accrue est plus importante que celle obtenue avec l'ATP seul montrant ainsi l'effet synergique de ces composés.

**Tableau 2 : libération de KGF par les cellules de la papille dermique issues de follicules pileux humains. Les résultats sont exprimés en valeurs moyennées des 3 donneurs ± sem (sem = écart standard à la moyenne).**

| | KGF (pg/ml) | sem | Comparaison, test Dunnett | |
|---|---|---|---|---|
| | | | Vs control | Vs Cressatine® |
| Contrôle | 26 | 4 | - | - |
| ATP | 37 | 5 | P<0,01 | - |
| Cressatine® | 32 | 9 | P=NS | - |
| ATP + Cressatine® | 48 | 11 | P<0,01 | P<0,01 |

| | | | | |
|---|---|---|---|---|
| Cressatine® : extrait aqueux de cresson et de capucine. | | | | |

En conclusion, l'ATP seul stimule la production de KGF, l'extrait aqueux de cresson et de capucine, permet d'augmenter la libération de HGF et l'association ATP et cressatine® stimule de façon synergique la production de HGF et de KGF. Ainsi l'effet synergique de ces 2 composés permet de stimuler la croissance du cheveu, de renforcer et de prolonger le cycle de vie des cheveux, de retarder et de ralentir la chute des cheveux et ainsi obtenir une chevelure plus couvrante.

### Exemple 2 : Exemple de composition triphasique selon l'invention :

| | |
|---|---|
| - Alcool | 10 à 40% |
| - Poudre (lactose) | 1 à 2% |
| - Emollients huileux | 10 à 20% |
| - Emollients aqueux | 1 à 5% |
| - Cresson (dans la phase aqueuse) | 0,01 à 0,5% |
| - Capucine (dans la phase aqueuse) | 0,01 à 0,5% |
| - ATP (dans la poudre) | 0,1 à 0,5% |
| - Colorant | < 0,1% |
| - Eau | qsp |

Les pourcentages exprimés correspondent à des % en poids par rapport à la composition totale.

Les trois phases sont mélangées extemporénément, l'ensemble est agité pour assurer la solubilisation des ingrédients pulvérulents dans la phase aqueuse ainsi qu'une émulsification des phases aqueuses et huileuse.

La composition obtenue est appliquée sur le cuir chevelu et n'est pas rincée.

## Revendications

1. Association comprenant un extrait aqueux de parties aériennes de cresson, un extrait aqueux de parties aériennes de capucine et de l'ATP.

2. Association selon la revendication 1, pour son utilisation dans la prévention et/ou le traitement de l'alopécie.

3. Association pour son utilisation selon la revendication 2 dans laquelle l'alopécie est choisie dans le groupe constitué par l'alopécie androgénétique, l'alopécie post-ménopausique, l'alopécie aiguë et l'alopécie aerata.

4. Utilisation cosmétique de l'association selon la revendication 1, pour promouvoir la croissance capillaire.

5. Composition cosmétique ou dermatologique comprenant à titre de principe actif anti-alopécie une association selon la revendication 1, avec au moins un excipient cosmétiquement ou dermatologiquement acceptable.

6. Composition selon la revendication 5, **caractérisée en ce qu'**elle comprend 0,01% à 0,5% en poids d'un extrait aqueux de cresson par rapport au poids total de la composition.

7. Composition selon l'une des revendications 5 ou 6, **caractérisée en ce qu'**elle comprend 0,01% à 0,5% en poids d'un extrait aqueux de capucine par rapport au poids total de la composition.

8. Composition selon l'une des revendications 5 à 7, **caractérisée en ce qu'**elle comprend 0,1% à 0,5% en poids d'ATP par rapport au poids total de la composition.

9. Composition selon l'une quelconque des revendications 5 à 8, **caractérisée en ce qu'**elle comprend en outre un autre principe actif anti-alopécie.

10. Composition selon l'une quelconque des revendications 5 à 9, **caractérisée en ce qu'**elle se présente sous une forme propre à une application topique.

11. Utilisation cosmétique d'une composition cosmétique selon l'une quelconque des revendications 5 à 10 pour promouvoir la croissance capillaire et/ou obtenir une chevelure plus couvrante.

12. Composition dermatologique selon l'une quelconque des revendications 5 à 10, pour son utilisation dans la prévention et/ou le traitement de l'alopécie.

13. Procédé de soin cosmétique des cheveux destiné à améliorer l'esthétique des cheveux en favorisant la pousse capillaire et/ou obtenir une chevelure plus couvrante, **caractérisé en ce qu'**il consiste à appliquer sur les cheveux et le cuir chevelu une quantité efficace d'une association comprenant un extrait de cresson, un extrait de capucine, et d'ATP selon la revendication 1 ou d'une composition cosmétique selon l'une des revendications 5 à 10, à laisser celle-ci au contact des cheveux, et éventuellement à rincer les cheveux.

## Patentansprüche

1. Verbindung, umfassend einen wässrigen Extrakt von oberirdischen Teilen von Kresse, einen wässrigen Extrakt von oberirdischen Teilen von Kapuzinerkresse und ATP.

2. Verbindung nach Anspruch 1 zu deren Verwendung bei der Verhinderung und/oder Behandlung von Alopezie.

3. Verbindung zu deren Verwendung nach Anspruch 2, wobei die Alopezie aus der Gruppe bestehend aus androgenetischer Alopezie, postmenopausaler Alopezie, akuter Alopezie und Alopecia areata ausgewählt ist.

4. Kosmetische Verwendung der Verbindung nach Anspruch 1 zum Fördern des Haarwuchses.

5. Kosmetische oder dermatologische Zusammensetzung, umfassend eine Verbindung nach Anspruch 1 als gegen Alopezie wirkenden Wirkstoff mit mindestens einem kosmetisch oder dermatologisch annehmbaren Hilfsstoff.

6. Zusammensetzung nach Anspruch 5, **dadurch gekennzeichnet, dass** sie 0,01 Gew.-% bis 0,5 Gew.-% eines wässrigen Extrakts von Kresse, bezogen auf das Gesamtgewicht der Zusammensetzung, umfasst.

7. Zusammensetzung nach einem der Ansprüche 5 oder 6, **dadurch gekennzeichnet, dass** sie 0,01 Gew.-% bis 0,5 Gew.-% eines wässrigen Extrakts von Kapuzinerkresse, bezogen auf das Gesamtgewicht der Zusammensetzung, umfasst.

8. Zusammensetzung nach einem der Ansprüche 5 bis 7, **dadurch gekennzeichnet, dass** sie 0,01 Gew.-% bis 0,5 Gew.-% ATP, bezogen auf das Gesamtgewicht der Zusammensetzung, umfasst.

9. Zusammensetzung nach einem der Ansprüche 5 bis 8, **dadurch gekennzeichnet, dass** sie ferner einen anderen gegen Alopezie wirkenden Wirkstoff umfasst.

10. Zusammensetzung nach einem der Ansprüche 5 bis 9, **dadurch gekennzeichnet, dass** sie in einer für eine topische Anwendung geeigneten Form dargereicht wird.

11. Kosmetische Verwendung einer kosmetischen Zusammensetzung nach einem der Ansprüche 5 bis 10 zum Fördern des Haarwuchses und/oder Erhalten eines volleren Haupthaars.

12. Dermatologische Zusammensetzung nach einem der Ansprüche 5 bis 10 zu deren Verwendung bei der Verhinderung und/oder Behandlung von Alopezie.

13. Verfahren zur kosmetischen Haarpflege, das zum Verbessern der Ästhetik von Har, indem das Haarwachstum unterstützt wird, und/oder Erhalten eines volleren Haupthaars vorgesehen ist, **dadurch gekennzeichnet, dass** es in dem Aufbringen einer wirksamen Menge einer Verbindung, umfassend einen Extrakt von Kresse, einen Extrakt von Kapuzinerkresse und ATP, nach Anspruch 1 oder einer kosmetischen Zusammensetzung nach einem der Ansprüche 5 bis 10 auf das Haar und die Kopfhaut, dem Belassen dieser in Kontakt mit dem Haar und eventuell dem Ausspülen des Haars besteht.

## Claims

1. A combination comprising an aqueous extract of watercress aerial parts, an aqueous extract of Indian cress aerial parts and ATP.

2. The combination according to claim 1, for its use in the prevention and/or treatment of alopecia.

3. The combination for its use according to claim 2, wherein the alopecia is selected from the group consisting of androgenetic alopecia, postmenopausal alopecia, acute alopecia, and alopecia areata.

4. Cosmetic use of the combination according to claim 1, to promote hair growth.

5. A cosmetic or dermatological composition comprising as anti-alopecia active ingredient a combination according to claim 1, with at least one cosmetically or dermatologically acceptable excipient.

6. The composition according to claim 5, **characterized in that** it comprises 0.01 to 0.5 wt% of an aqueous extract of watercress relative to the total weight of the composition.

7. The composition according to one of claims 5 or 6, **characterized in that** it comprises 0.01 to 0.5 wt% of an aqueous extract of Indian cress relative to the total weight of the composition.

8. The composition according to one of claims 5 to 7, **characterized in that** it comprises 0.1 to 0.5 wt% of ATP relative to the total weight of the composition.

9. The composition according to any one of claims 5 to 8, **characterized in that** it further comprises another anti-alopecia active ingredient.

10. The composition according to any one of claims 5 to 9, **characterized in that** it comes in a form suitable for topical application.

11. Cosmetic use of a cosmetic composition according to any one of claims 5 to 10 to promote hair growth and/or to obtain a head of hair which provides greater coverage.

12. The dermatological composition according to any one of claims 5 to 10, for its use in the prevention and/or treatment of alopecia.

13. A process for cosmetic care of the hair intended to improve the aesthetic quality of the hair by promoting hair growth and/or to obtain a head of hair which provides greater coverage, **characterized in that** it consists in applying to the hair and the scalp an effective amount of a combination comprising an extract of watercress, an extract of Indian cress, and ATP according to claim 1 or of a cosmetic composition according to one of claims 5 to 10, in leaving same in contact with the hair, and optionally in rinsing the hair.
